# EUROPEAN PATENT APPLICATION

(11) **EP 3 839 970 A1**
(43) Date of publication of application: **23.06.2021**
(21) Application number: 19217322.7
(22) Date of filing: 18.12.2019
(51) Int. Cl.: G16H 50/20, G16H 50/30

(54) **ESTIMATING THE RISK OF A SUBJECT REACHING A PARTICULAR MEDICAL OUTCOME**

(71) Applicant: Koninklijke Philips N.V., 5656 AG Eindhoven (NL)
(72) Inventor: DEN BRINKER, Albertus Cornelis, 5656 AE Eindhoven (NL); KLEE, Mareike, 5656 AE Eindhoven (NL)
(74) Representative: Philips Intellectual Property & Standards

(57) **Abstract**

A system provides an estimate of the risk of a subject reaching a particular medical outcome. A set of medical outcome models is used, each providing risk estimate and an accuracy indication. Different models use different sets of patient information parameters as their input so that different models are appropriate for different available patient information. A medical outcome model is used which can operate with the available patient information parameters and which has a best accuracy indication. This provides flexibility for operating with different users, and the system can evolve as the models evolve over time.

## Description

### FIELD OF THE INVENTION

This invention relates to the task of estimating the risk of a subject reaching a particular medical outcome, such as the risk of a subject needing re-admission to hospital.

### BACKGROUND OF THE INVENTION

To risk-stratify patients is to rank patients according to the risk of an adverse event. This can be on a continuous scale (such as 0 to 100) or it can base on categories such moderate health risk and low health risk tiers. Risk stratification is used to align the very limited time and resources of a medical practice with the needs of its patient population. It typically involves sophisticated algorithms and robust registries, but it also relies on patient experience and physician judgment.

Various risk stratification algorithms exist. They differ in the addressed risk, the addressed features (prediction variables), and the data origins (geographic location, medical site and population).

For instance, an example of a possible risk estimate is the risk that a COPD patient will be re-admitted to hospital in the next year. Of course, any other period may be considered, such as half a year or two years. The risk may focus on re-admittance associated with the COPD condition, or it may relate to the risk of re-admittance with any potential cause.

Existing risk-stratification models are rather rigid in their operation. They are trained and optimized under certain restrictive conditions (e.g. for specific populations, certain feature sets, certain risks). This makes it difficult to transfer the system from one case to the next or, even worse, the system may be limited due to the fact that not all data needed to drive the model is available for a specific patient.

There is therefore a need for a system and method for risk assessment that is sufficiently flexible to cope with numerous situations which occur in practice.

### SUMMARY OF THE INVENTION

The invention is defined by the claims.

According to examples in accordance with an aspect of the invention, there is provided a system for providing an estimate of the risk of a subject reaching a particular medical outcome, comprising:
a set of medical outcome models, each model comprising:
   an input for receiving a respective set of patient information parameters;
   an estimation algorithm; and
   an output for providing:
an estimate of the risk of a subject reaching the particular medical outcome; and
an indication of the accuracy of the estimate,
wherein different medical outcome models in the set have different sets of patient information parameters as their input; and
a processor which is adapted to:
   receive available patient information parameters;
   select the medical outcome model which has as input the available patient information parameters or a sub-set thereof, and which has a best indication of the accuracy of the estimate;
   apply the selected medical outcome model to the available patient information parameters; and
   output the estimate of the risk and the indication of the accuracy of the estimate.

This system makes use of a multiple models. The different models make use of different sub-sets of possible patient information parameters, and as a result they have different levels of accuracy in performing the risk estimation. For a given available set of patient information parameters, there will generally be a sub-set of the models which can be used. From that sub-set, the one with the best accuracy is chosen.

Thus, the system makes use of a set of models so that the available patient information can be handled in the most effective way, to reach the most accurate possible estimate of the patient reaching the medical outcome being tested.

The different sets of patient information parameters together form global patient data. This global patient data is used previously to create the different models, for example based on machine learning from a database of patient information.

The indication of the accuracy of the estimate comprises for example a grade from a set of possible grades. This provides an easy to understand accuracy indication.

The processor may be further adapted to indicate the additional patient information parameters that would be needed to achieve a better accuracy of the estimate by using a different model.

In this way, the user of the system may be prompted to obtain additional patient information parameters so that a better accuracy can be achieved.

The particular medical outcome is for example the need for re-admittance to hospital before a certain point of time in future. The system thus provides an indication of whether patient symptoms are likely to deteriorate to such an extent that hospital re-admission will be needed.

The certain point in time may be a number of months or years ahead.

The particular medical outcome is for example the risk of re-admittance to hospital of a COPD patient.

The patient information parameters may comprise patient physiology characteristics and patient medical data. The physiology characteristics for example comprise age, height, gender, weight. BMI, etc. The patient medical data for example comprises medications being taken, or co-morbidities. Psycho-social factors and socio-economic factors may also be included.

The invention also provides an arrangement for providing an estimate of the risk of a subject reaching a selected medical outcome from a set of possible medical outcomes, comprising:
the system as defined above for each of the possible medical outcomes;
an input for receiving the selected medical outcome; and
a selector for selecting the system based on the selected medical outcome.

Thus, an arrangement may enable testing of multiple possible medical outcomes.

The invention also provides a computer-implemented method for providing an estimate of the risk of a subject reaching a particular medical outcome, comprising:
receiving available patient information parameters;
selecting a medical outcome model from a set of medical outcome models, each model comprising:
   an input for receiving a respective set of patient information parameters;
   an estimation algorithm; and
   an output for providing an estimate of the risk of a subject reaching the particular medical outcome and an indication of the accuracy of the estimate,
   wherein different medical outcome models in the set have different sets of patient information parameters as their input,
applying the selected medical outcome model to the available patient information parameters; and
outputting the estimate of the risk and the indication of the accuracy of the estimate,
wherein the selecting comprises determining which medical outcome model has as input the available patient information parameters or a sub-set thereof and which has the best indication of the accuracy of the estimate.

This method uses of multiple models so that different sub-sets of possible patient information parameters may be used as model inputs. The different models have different levels of accuracy in performing the risk estimation. From the sub-set of models which can be used, the one with the best accuracy is chosen.

The indication of the accuracy of the estimate for example comprises a grade from a set of possible grades. The additional patient information parameters that would be needed to achieve a better accuracy of the estimate by using a different model may also be indicated.

The patient information parameters comprises patient physiology characteristics and patient medical data.

The method of the invention may be implemented in software.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a system for providing an estimate of the risk of a subject reaching a particular medical outcome;
Figure 2 shows a system for creating the models used in the system of Figure 1; and
Figure 3 shows a method for providing an estimate of the risk of a subject reaching a particular medical outcome.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a system which provides an estimate of the risk of a subject reaching a particular medical outcome. A set of medical outcome models is used, each providing a risk estimate and an accuracy indication. Different models use different sets of patient information parameters as their input so that different models are appropriate for different available patient information. A medical outcome model is used which can operate with the available patient information parameters and which has a best accuracy indication. This provides flexibility for operating with different users, and the system can evolve as the models evolve over time.

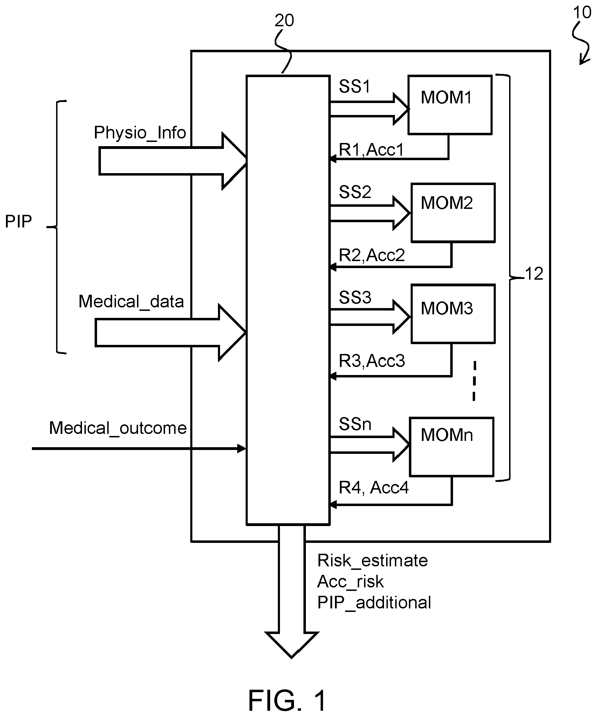

Figure 1 shows a system 10 for providing an estimate of the risk ("Risk_estimate") of a subject reaching a particular medical outcome.

The system has a set 12 of medical outcome models MOM1 - MOMn. Each model has an input for receiving a respective set of patient information parameters.

A set of patient information parameters PIP is provided as input to the system. The patient information parameters for example comprise patient physiology characteristics "Physio info" and patient medical data "Medical_data".

The physiology characteristics for example comprise age, height, gender, weight. BMI, etc. The patient medical data for example comprise medications being taken, or co-morbidities. Psycho-social factors and socio-economic factors may also be included.

There is a full possible set of patient information parameters (on which the models are based) which may be termed patient data.

The input to each model MOM1 - MOMn is a sub-set SS1 - SSn of this patient data (i.e. a sub-set of the full set of patient information parameters). One of the medical outcome models could however receive as input the full set of patient information parameters, i.e. the full patient data, but typically the models only use a reduced sub-set of the patient data. Different medical outcome models in the set have different sets of patient information parameters as their input, i.e. the sub-sets are different. Thus, different models are available for use according to the patient information parameters PIP that are available.

Each medical outcome model comprises an estimation algorithm for providing a respective risk estimate RE1 - REn and an associated indication Acc1 - Accn of the accuracy of that risk estimate.

A processor 20 receives the available patient information parameters PIP. From this, it determines which medical outcome models MOM1 - MOMn have as input the available patient information parameters or a sub-set thereof. In other words, patient information parameters PIP are received as input. The parameters enable inputs to be generated for some, but typically not all, of the medical outcome models. For example, the patient information parameters PIP may include all the parameters of the sub-set SS1. Thus, the model MOM1 may be used, because the required set of inputs to that model can be generated. However, the patient information parameters PIP may miss a required input of the sub-set SS2 so model MOM2 may not be used.

It may be possible to estimate some parameters to enable additional models to be used although there may be a consequent accuracy sacrifice by using estimated parameters.

For all of the models that are available for use (either with real data or with some estimated data), the model is selected which has the best indication of the accuracy of the estimate.

The selected medical outcome model is then applied to the available patient information parameters PIP which have been received as input.

The system then outputs the estimate of the risk "Risk_estimate" and the indication of the accuracy of the estimate "Acc_risk".

The different models have different levels of accuracy in performing the risk estimation. The use of a set of models means the available patient information can be handled in the most effective way, to reach the most accurate possible estimate of the patient reaching the medical outcome being tested.

The indication of the accuracy of the estimate comprises for example a grade from a set of possible grades. This provides an easy to understand accuracy indication.

Figure 1 also shows an output from the system in the form of an indication of the additional patient information parameters "PIP_additional" that would be needed to achieve a better accuracy of the estimate by using a different model. In this way, the user of the system may be prompted to obtain additional patient information parameters so that a better accuracy can be achieved.

As mentioned above, one possible medical outcome to be tested is the need for re-admittance to hospital before a certain point of time in future, such as six months, or a year, or two years, etc.. The system thus provides an indication of whether patient symptoms are likely to deteriorate to such an extent that hospital re-admission will be needed. This is for example of interest for COPD patients.

It may be that the patient information parameters are too limited for any of the models to be used. In this case, the output may then indicate that no risk estimate is available. In such a case, an indication can also be provided of options to supplement the patient information parameters such that at least a minimal accuracy model estimate may be obtained. For example, the system could indicate that BMI or age is additionally needed in order to be able to create an estimate. Similarly, if at low accuracy, suggestion(s) could be provided concerning which additional data would be required to create a higher-accuracy estimate.

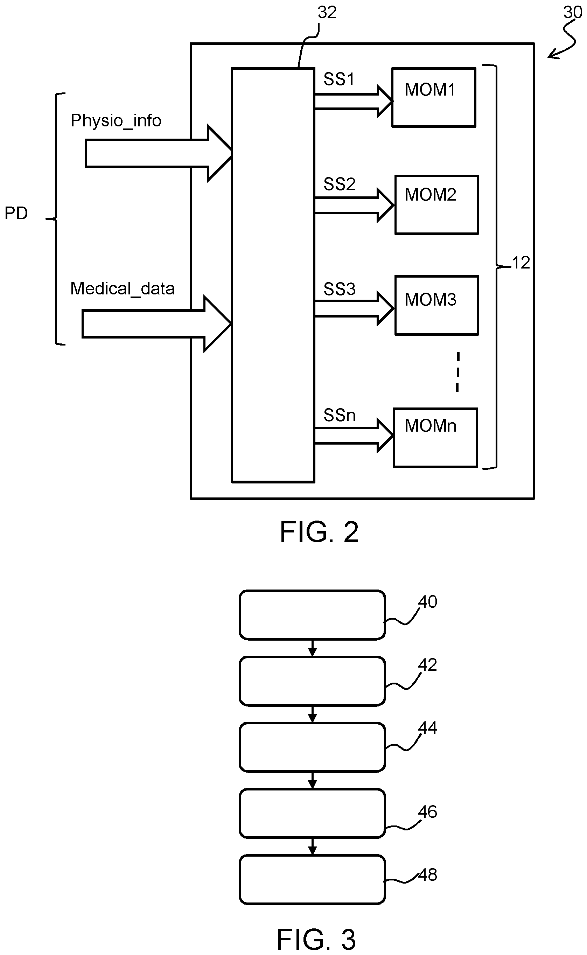

Figure 2 is used to show a 30 system for generating the medical outcome models MOM1 - MOMn. Figure 2 shows a model creation unit 32 forming a processor within the system. It receives patient data PD as historical data for a large number of patients, as well as the patient outcomes "Medical_outcomes".

The patient data PD and patient outcomes together are obtained from a historical patient database consisting of patient demographics, medical history, etc.

A machine learning algorithm or set of algorithms generates the set of medical output models MOM1 - MOMn, for each considered variable (i.e. each particular medical outcome to be modeled).

The models are characterized by the predicted variable (i.e. the medical outcome being modeled), the required inputs, the model parameters and the model accuracy. This model information is used to organize the set of models (per considered variable) according to accuracy levels. Thus, for each variable (e.g. the risk of hospital re-admission within one year) there is a set of models for generating an estimate of this risk, based on an available set of patient information parameters (which is a respective sub-set of the full patient data).

For each variable, multiple models are generated, where the models differ in the required inputs. This sorting of models may also remove models as being too inaccurate, or it may remove models deemed superfluous for achieving certain accuracy ranges, e.g. because other models already achieve the same accuracy with a smaller (or more readily available) set of input patient information parameters. A model is superfluous at least if within a certain accuracy class another model exists having as input a sub-set of parameters of the considered model.

The models are thus organized by the model creation unit 32, and an accuracy indicator is provided. The model creation unit 32 also determines what additional patient information parameters may be needed for a different model with a better accuracy level band to be available for use. If there are models requiring a minimal additional input, this information is used to provide advice to the user, as mentioned above.

By way of example, the variable may be the risk for a discharged COPD patient to be readmitted to the hospital within one year. Historical data is used for the model creation, using machine learning, whereby the outcome of interest (e.g. re-admittance to the hospital within one year) is available together with the patient data at or before the observation period (i.e., the one-year re-admittance period) or estimates of this patient data may be generated. The model can then be built to predict the risk (e.g. of re-admittance) based on the patient data.

This model may use a multitude of parameters such as patient characteristics (age, height, etc.), medical data (like exacerbation frequency in the last year, medication usage, number of co-morbidities, etc.), psycho-social determinants, and socio-economic factors. The accuracy measure is for example an accuracy of the prediction, or a goodness-of-fit.

Not all input parameters are equally relevant but there are certain combinations of input parameters such that an acceptable degree of accuracy is reached. The acceptable degree of accuracy is typically defined in terms of over- or under-fitting of data. This presumes that the uncertainty in the data is known or can be estimated and that model predictions can be evaluated relative to this intrinsic uncertainty.

Thus, from the full set of patient information parameters, i.e. the full patient data, several sub-sets can be defined which yield, with possible different models or different parametrizations in the same model, an acceptable prediction quality.

Each model is generated using historical data from multiple patients, whereby the patient data has been pooled into sub-sets to characterize sub-sets of patients which are identical according to certain criteria, e.g. disease severity, age, gender. There are multiple patients within each sub-set and thus there is an observed distribution of outcomes. The range of outcomes may determine a quality indicator and hence the associated accuracy.

Another accuracy and quality characterization for each model could be based on correlation levels or agreement metrics (e.g. Cohen's kappa). The model predictions may for example be associated with one of these metrics and a certain range (e.g. correlation between 0.5 and 1) may then be subdivided into interval ranges.

The model construction process may be performed on an ongoing basis. Each accuracy category is populated with as many models as possible having different sub-sets of parameters. The models may thus be adapted (based on the machine learning nature of the algorithm used to generate the models) because the database of patient data will be evolving over time. Models may be adapted, or be discarded or come into existence, over time.

Regardless of the model being used, the user is provided an indication of the accuracy (or inaccuracy) of the model, thereby allowing this continuous flux of models without the need for tracing which exact model was used.

The invention has been described above in general terms. A more concrete example will now be presented.

An example of the full set of patient information parameters, i.e. the patient data, is:
Age
Weight
Height
BMI: Body Mass Index
Pack_Years: amount of years of smoking
ExacF: exacerbation frequency of the medical condition being assessed in the last year
AdmF: admission frequency in the last year
FEV1: Forced Expiratory Volume in 1 second
FEV1%Pred: predicted FEV1% (which is the FEV1 as a percentage of the forced vital capacity; a lung function test parameter
GOLD: COPD severity stage
6MWD: 6-minute walking distance test
HADSa: Hospital Anxiety and Depression Scale anxiety score
HADSd: Hospital Anxiety and Depression Scale depression score
HADStot: total HADS score
MediUse: Indicator of the amount of medication used
CoMorb: Comorbidity index

As an example, the risk prediction may be a number between 0 and 1 for a patient. Taking the example as above, the risk could be the probability that the patient will be admitted to hospital for COPD treatment in the next year.

A patient database for example includes information relating to the observed risk as well as all the parameters above for multiple patients. Machine learning is then used to approximate the observed risk based on the factors above, and for different combination of the patient information parameters.

The best approximation produces an overall or average difference, termed the loss. There are many ways to define the loss, for example as a numeric value larger than 0. Four model categories may for example be defined:
Category A is the category containing models with a loss less than 1.1 (best models).
Category B is the category of models with loss larger or equal than 1.1 but less than 1.3.
Category C is the category of models with loss larger or equal than 1.3 but less than 1.8.
Category D is the category of models with loss larger or equal to 1.8; this is the category of models with insufficient accuracy, and its estimates are not used as output.

Modelling the risk data, it may for example be found that using the admission frequency AdmF, the exacerbation frequency ExacF, the FEV1 and the 6MWD, a model is obtained with loss 0.9, i.e. in category A.

If the input parameter 6MWD is omitted, a model may be obtained with loss 1.17 thus in category B.

If the input data (AdmF, ExacF and FEV1) is then augmented with HADSd, BMI, MediUse, and CoMorb Log, a model may again be available in Category A.

Similarly, if FEV1 is omitted, and FEV1_pred is used instead, the resulting model may still remain in the category A.

If ExacF is omitted, it may for example only be possible to attain at best a model in category C, for instance by using AdmF, FEV1 and 6MWD.

If AdmF is omitted, it may for example only be possible to create a model in category D: essentially lacking data to create any reliable model.

This illustrates that different choices (or availability) of patient information parameters lead to models of different quality. Some factors can be traded for others to attain the same level of quality, and some factors are required to attain a minimal degree of accuracy.

As mentioned above, the medical outcome models all relate to a particular medical outcome. However, the system may have multiple sets of models, each for a different medical outcome.

There is then an arrangement for providing an estimate of the risk of a subject reaching a selected medical outcome from a set of possible medical outcomes. The system describe above is applicable separately to each of the possible medical outcomes. As shown in Figure 1, the system 10 then has an input "Medical_outcome" for identifying a selected medical outcome. The processor 20 then operates as a selector to select the system (i.e. the set of models) based on the selected medical outcome.

Figure 3 shows a computer-implemented method for providing an estimate of the risk of a subject reaching a particular medical outcome.

In step 40, available patient information parameters are received, and an indication of the medical outcome being analyzed (if the system is capable of analysis of multiple medical outcomes).

In step 42, a medical outcome model is selected from a set of medical outcome models for that particular medical outcome. The selection is based on the accuracy indicator.

In step 44, the selected medical outcome model is applied to the available patient information parameters.

In step 46, the estimate of the risk is output as well as an indication of the accuracy of the estimate.

In step 48, the user may be presented with additional information to advise of additional patient information parameters that could be used to improve the accuracy of the estimate.

The individual models may be generated based on machine learning algorithms, as mentioned above.

A machine-learning algorithm is any self-training algorithm that processes input data in order to produce or predict output data. Here, the input data (for each medical outcome being considered) comprises patient information parameters and the associated medical outcomes for a database of patients with those parameters, and the output data comprises the estimate of the risk of the particular medical output for that set of patients.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

As discussed above, the system makes use of processor to perform the data processing (both in the system as used, and during creation of the models). The skilled person would be readily capable of developing a processor for carrying out any herein described method. Thus, each step of a flow chart may represent a different action performed by a processor, and may be performed by a respective module of the processing processor.

The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

The invention may be applied to any medical outcome and is not limited to hospital re-admission. Any medical outcome, preferably with a binary (yes/no) property may be modeled using machine learning from historical datasets. Examples are disease reappearance (i.e. end of remission) within a certain time, death within a certain time, a certain definable level of disease progression within a certain time, disease reappearance within the lifetime of the patient, etc.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A system (10) for providing an estimate of the risk of a subject reaching a particular medical outcome, comprising:
a set (12) of medical outcome models (MOM1-MOMn), each model comprising:
an input for receiving a respective set (SS1-SSn) of patient information parameters;
an estimation algorithm; and
an output for providing:
an estimate of the risk (RE1-REn) of a subject reaching the particular medical outcome; and
an indication of the accuracy of the estimate (Acc1-Accn),
wherein different medical outcome models in the set have different sets of patient information parameters as their input; and
a processor (20) which is adapted to:
receive available patient information parameters (PIP);
select the medical outcome model (MOM1-MOMn) which has as input the available patient information parameters or a sub-set thereof, and which has a best indication of the accuracy of the estimate (Acc1-Accn);
apply the selected medical outcome model to the available patient information parameters; and
output the estimate of the risk (Risk_Estimate) and the indication of the accuracy of the estimate (Acc_risk).

2. The system as claimed in claim 1, wherein the indication of the accuracy of the estimate (Acc_risk) comprises a grade from a set of possible grades.

3. The system as claimed in claim 1 or 2, wherein the processor (20) is further adapted to indicate the additional patient information parameters (PIP_additional) that would be needed to achieve a better accuracy of the estimate by using a different model.

4. The system as claimed in any one of claims 1 to 3, wherein the particular medical outcome is the need for re-admittance to hospital before a certain point of time in future.

5. The system as claimed in claim 4, wherein the certain point in time is a number of months or years ahead.

6. The system as claimed in claim 4 or 5, wherein the particular medical outcome is the risk of re-admittance to hospital of a COPD patient.

7. The system as claimed in any one of claims 1 to 6, wherein the patient information parameters (PIP) comprises patient physiology characteristics (Physio_info) and patient medical data (Medical_data).

8. An arrangement for providing an estimate of the risk of a subject reaching a selected medical outcome from a set of possible medical outcomes, comprising:
a system as claimed in any one of claims 1 to 7 for each of the possible medical outcomes;
an input for receiving the selected medical outcome; and
a selector for selecting the system based on the selected medical outcome.

9. A computer-implemented method for providing an estimate of the risk of a subject reaching a particular medical outcome, comprising:
(40) receiving available patient information parameters;
(42) selecting a medical outcome model from a set of medical outcome models, each model comprising:
an input for receiving a respective set of patient information parameters;
an estimation algorithm; and
an output for providing an estimate of the risk of a subject reaching the particular medical outcome and an indication of the accuracy of the estimate,
wherein different medical outcome models in the set have different sets of patient information parameters as their input,
(44) applying the selected medical outcome model to the available patient information parameters; and
(46) outputting the estimate of the risk and the indication of the accuracy of the estimate,
wherein the selecting comprises determining which medical outcome model has as input the available patient information parameters or a sub-set thereof and which has the best indication of the accuracy of the estimate;

10. The method as claimed in claim 9, wherein the indication of the accuracy of the estimate comprises a grade from a set of possible grades.

11. The method as claimed in claim 9 or 10, further comprising indicating (48) the additional patient information parameters that would be needed to achieve a better accuracy of the estimate by using a different model.

12. The method as claimed in any one of claims 9 to 11, wherein the particular medical outcome is the need for re-admittance to hospital before a certain point of time in future.

13. The method as claimed in claim 12, wherein the particular medical outcome is the risk of re-admittance to hospital of a COPD patient.

14. The method as claimed in any one of claims 9 to 13, wherein the patient information parameters comprises patient physiology characteristics and patient medical data.

15. A computer program comprising computer program code means which is adapted, when said program is run on a computer, to implement the method of any one of claims 9 to 14.
